# EUROPEAN PATENT SPECIFICATION

(11) **EP 0 577 206 B1**
(45) Date of publication and mention of the grant of the patent: **26.08.1998**
(21) Application number: 93201844.3
(22) Date of filing: 25.06.1993
(51) Int. Cl.: C07D 305/12, C08G 63/08, C07D 315/00

(54) **Carbonylation of epoxides**
Carbonylierung von Epoxyden
Carbonylation d'époxydes

(30) Priority: 29.06.1992 EP 92201940
(43) Date of publication of application: 05.01.1994
(73) Proprietor: SHELL INTERNATIONALE RESEARCH MAATSCHAPPIJ B.V., 2596 HR Den Haag (NL)
(72) Inventor: Drent, Eit, NL-1031 CM Amsterdam (NL); Kragtwijk, Eric, NL-1031 CM Amsterdam (NL)

(56) References cited:
- DE-A- 2 901 347
- GB-A- 1 020 575
- US-A- 2 782 226
- US-A- 3 260 738

## Description

This invention relates to a process for the carbonylation of epoxides using a cobalt catalyst.

It is known that epoxides may be carbonylated by reaction with carbon monoxide. The primary product of the carbonylation reaction is a β-propiolactone formed by insertion of a carbonyl group into a carbon oxygen bond of the oxirane group of the epoxide. Secondary products, such as β-propiolactone polymers, 3-hydroxycarboxylic acids and esters, and acrylic acids and esters, may also be formed directly or indirectly under specific conditions due to the reactive nature of the primary β-propiolactone product.

According to GB-A-1020575 the carbonylation reaction requires the presence of a catalyst, preferably a cobalt catalyst such as a cobalt carbonyl. Using ethylene oxide as substrate, β-propiolactone is obtained as primary product, whereas the presence of metal or quaternary ammonium halides promotes the formation of β-propiolactone polymer. The tendency of 1,2-epoxides to homopolymerise may be diminished by adding a small proportion of a base, such as pyridine, to the reaction mixture.

A process for the preparation of a 3-hydroxypropionate ester by conducting the carbonylation in the presence of an alcohol is disclosed by US-A-3260738. This process uses a cobalt carbonyl catalyst, which preferably is modified by a tertiary phosphine or tertiary amine co-catalyst, including N-heterocycles such as pyridine and methylpyridine. A similar process is disclosed by Y. Kawabata, et al., in Nippon Kagaku Kaishi, (5), 635-40 (1979).

US-A-2782226 discloses a process wherein the carbonylation is conducted in the presence of water. The ethylene glycol monoester of 3-hydroxypropionic acid as end product is obtained through the addition of a further ethylene oxide molecule to the intermediate 3-hydroxypropionic acid reaction product. Cobalt on kieselguhr is used as catalyst.

US-A-4209467 discloses catalyst systems comprising cobalt and a hydroxy substituted pyridine compound, which are used in the hydroformylation of olefins to aldehydes.

Whereas the prior art shows the feasibility of the carbonylation technology for preparing the β-propiolactones and derivatives, the exemplified catalyst systems leave room for improvement in terms of achievable selectivity and reaction rate in order to arrive at an industrially competitive process. The present invention aims at providing such improvement.

A substantially improved process has been found for the carbonylation of epoxides by reaction with carbon monoxide at elevated pressure and temperature in the presence of a catalyst system comprising a source of cobalt and a hydroxy substituted pyridine compound. As compared with the previously used unsubstituted pyridine, the present invention provides a much higher selectivity to the β-propiolactone product. When using a preferred 3-hydroxy substituted pyridine compound, the carbonylation reaction additionally proceeds at a dramatically increased rate.

The metal component of the catalyst system to be used according to the invention is a conventional cobalt catalyst. It may be introduced into the reaction in the form of any source of cobalt, which is converted into the catalytically active species under carbon monoxide pressure applied in the process. A very suitable source of cobalt is constituted by dicobalt octacarbonyl and/or other cobalt carbonyls. Cobalt salts, such as cobalt acetate may be used as catalyst precursor, as they will be readily carbonylated under the carbon monoxide pressure. When adding finely-divided cobalt metal to the reaction mixture for preparing cobalt carbonyls in situ, careful control of temperature will be required for avoiding undesired side reactions. Generally, the cobalt catalyst or precursor will be soluble in the reaction medium. However, the catalyst may also be used in a heterogenised form, what would allow for a gaseous reaction medium. The cobalt source may be added in the form of a pre-formed complex with the required pyridine compound.

The further component of the present catalyst system is a pyridine compound carrying at least one hydroxy substituent. The expression "pyridine compound" is meant here to encompass any compound comprising the pyridine nucleus, i.e. a six-membered heteroaromatic ring containing an imino nitrogen atom. Therefore, multi-ring compounds such as quinoline and 4,4'-bipyridyl can be used when having a hydroxy substituent. Besides the essential hydroxy substituent, the pyridine ring may carry further substituents, which do not interfere with the carbonylation reaction.

Representative examples of suitable hydroxy substituted pyridine compounds include 2-hydroxypyridine, 3-hydroxypyridine, 4-hydroxypyridine, 3,4-dihydroxypyridine, 3-hydroxyquinoline, 4-hydroxy-2-methylpyridine, and 3-hydroxy-4-methylpyridine. The best results were obtained using 3-hydroxy substituted pyridine compounds, which therefore are preferred, in particular 3-hydroxypyridine.

The ratio of hydroxy substituted pyridine compound to cobalt in the present catalyst systems is not critical and may vary within wide limits. Practical ratios are in the range of from 0.5 to 20 moles of hydroxy substituted pyridine compound per gram atom of cobalt, with ratios in the range of from 1 to 5 moles of hydroxy substituted pyridine compound per gram atom of cobalt being preferred.

The substrates being subjected to carbonylation in accordance with the present invention are epoxides as derivable by addition of an oxygen atom to an olefinic double bond to form an oxirane ring. The oxirane ring may be substituted with alkyl groups, as in propylene oxide, or other groups, as in styrene oxide. Preferably, the epoxide substrate is an 1,2-epoxide, in particular an optionally substituted 1,2-epoxyalkane. Representative epoxides include ethylene oxide, propylene oxide, styrene oxide, 1,2-epoxyhexane, and 1,2-epoxyoctane.

When the epoxide is reacted with carbon monoxide in the absence of further reactants, the primary product is a β-propiolactone, which may be substituted at the α- and/or β-position, if the starting epoxide constitutes a substituted oxirane. For example, β-butyrolactone [β-methyl-β-propiolactone or 2-oxo-4-methyloxetane] is obtained as product of the carbonylation of propylene oxide, whereas β-propiolactone is the product of the carbonylation of ethylene oxide. Due to the tendency of β-propiolactones to polymerise, the reaction mixture may contain a certain proportion of β-propiolactone polymer, the amount of which slowly increases upon standing. If desired, the direct yield of polymer may be increased by the addition of promoters to the reaction system. Examples of such promoters are metal halides and quaternary ammonium halides, which may be used in a proportion of from 0.10 to 5.0 %wt of the epoxide.

The carbonylation reaction is conveniently conducted in the liquid phase. If the epoxide substrate and lactone product are liquid, there is no need for using a separate solvent. However, the use of a separate solvent may be convenient and preferred for practical purposes, for example in connection with product recovery. Suitable solvents include moderately polar aprotic solvents, in particular ethers such as diglyme (2,5,8-trioxanonane), diphenylether, THF and anisole. Protic solvents such as alcohols are usable, but may participate in the reaction to yield other products.

The temperature and pressure for the carbonylation reaction are not critical and may vary within wide limits. It is an advantageous feature of the invention that the reaction may be conducted at mild conditions. Very suitable temperatures are in the range of from 50 to 150 °C, more suitably from 60 to 100 °C. At lower temperature the reaction may be unduly retarded, whereas higher temperatures may induce the formation of secondary derivatives. Typical pressures are below 150 bar, as higher pressures would involve excessive equipment cost. Preferred pressures are in the range of 20 - 100 bar.

The β-propiolactone product can be recovered as such using conventional separation technology. It may also be recovered in the form of its polymer. Comprising a strained four-membered ring, the optionally substituted β-propiolactones obtained readily undergo ring cleavage reactions. They may thermally polymerise to form polyesters of the AB-type. Reaction with alcohols under acidic conditions provides β-alkoxy carboxylic acids, whereas under alkaline conditions β-hydroxy carboxylic esters are obtained. By the use of sodium alkoxylates as alkaline material β-alkoxy carboxylic esters are directly accessible. Reaction with water provides β-hydroxy carboxylic acids.

Moreover, the β-propiolactones, particularly when substituted at the β-position, or their polymers, show a marked tendency to dehydration to yield acrylic type of products. For example, it is known that β-lactone polymers on pyrolysis at temperatures of from about 180 to 250 °C in the presence of a polymerisation inhibitor give good yields of α,β-unsaturated carboxylic acids. When heated with an alcohol in the presence of a dehydrating agent, α,β-unsaturated esters can be made.

Accordingly, the present β-propiolactone products constitute versatile precursors to various β-propiolactone derivatives, which in turn are useful and find application as chemical solvent or as intermediate for further chemical derivatives or polymers.

According to an advantageous feature of the present invention, such derived products may directly be produced by conducting the present carbonylation in the presence of a suitable coreactant such as a hydroxy compound. For example, when using an alcohol as coreactant, the product of the carbonylation reaction is a β-hydroxy carboxylic ester, and using water as coreactant a β-hydroxy carboxylic acid is obtained. For example, the reaction of ethylene oxide with carbon monoxide and methanol in the presence of a catalyst system comprising cobalt carbonyl and 3-hydroxypyridine yields the methyl ester of 3-hydroxypropionic acid with excellent selectivity. It will be appreciated that a coreactant such as an alcohol may also act as the solvent for the reaction.

The invention will be further illustrated by the following examples.

### Examples 1-4 and Comparative Example A

Each time a 250 ml stainless-steel autoclave was charged with 10 ml ethylene oxide, 40 ml diglyme, 1 mmol dicobalt octacarbonyl [Co₂(CO)₈] and 4 mmol of a pyridine compound as indicated, under exclusion of ambient oxygen. Then the autoclave was pressurised with 60 bar of carbon monoxide and heated for the indicated periods at the indicated temperatures. At the end of the reaction period, the autoclave was cooled and a sample of its content was analysed by standard gas liquid chromatography (GLC) and ¹³C NMR. The results of the experiments are summarised in Table 1 below, wherein the conversion is expressed as the percentage of ethylene oxide being converted, the selectivity represents the molar percentage of β-propiolactone formed relative to the total amount of converted ethylene oxide, and the turnovers represent the reaction rate expressed as mol of ethylene oxide converted per gram atom of cobalt per hour.

**Table 1**

| Ex. No. | pyridine compound | t (h) | T (°C) | conversion,(%) | selectivity*) (%) | turnovers (mol/gr at Co/hour) |
|---|---|---|---|---|---|---|
| 1 | 3-hydroxypyridine | 3 | 60 | 100 | > 98**) | 33 |
| 2 | 3-hydroxypyridine | 1 | 75 | 100 | > 98 | 100 |
| 3 | 3-hydroxypyridine | 0.5 | 80 | 100 | > 98 | 200 |
| 4 | 4-hydroxypyridine | 10 | 75 | 81 | > 98 | 10 |
| A (comp) | pyridine | 6 | 75 | 33 | 50 | 8 |

| | | | | | | |
|---|---|---|---|---|---|---|
| *) on standing at room temperature the initially formed lactone, as determined by ¹³C NMR, is slowly converted to polyhydroxypropionate; on injection in GLC pyrolysis to acrylic acid takes place. | | | | | | |
| **) trace of aldehyde. | | | | | | |

It is seen that the selectivity of the carbonylation reaction is much increased by the use of a catalyst system as proposed by the present invention when compared with the use of pyridine according to the prior teaching. It is also seen that the preferred use of a 3-hydroxy substituted pyridine compound concomitantly enhances the reaction rate when comparing examples 2, 4 and A conducted at the same temperature.

### Example 5

Using the general procedure of Example 1, an autoclave was charged with 20 ml propylene oxide, 40 ml diglyme, 2 mmol of dicobalt octacarbonyl and 4 mmol of 3-hydroxypyridine, and pressurised with 60 bar carbon monoxide. After heating for 6 hours at 75 °C, 93 % conversion of propylene oxide was observed with a selectivity of > 90 % into β-butyrolactone (2-oxo-4-methyloxetane).

### Example 6

Using the general procedure of Example 1, an autoclave was charged with 10 ml isobutylene oxide, 40 ml diglyme, 2 mmol of dicobalt octacarbonyl and 4 mmol of 3-hydroxypyridine, and pressurised to 60 bar carbon monoxide. After heating for 10 hours at 75 °C, 60 % conversion of isobutylene oxide was observed into 3-hydroxy-3-methylbutanoic acid lactone (2-oxo-4,4-dimethyloxetane) according to ¹³C NMR, which was pyrolysed into 3-methyl-2-butenoic acid upon injection into GLC.

### Example 7

Using the general procedure of Example 1, an autoclave was charged with 30 ml ethylene oxide, 100 ml diglyme, 2 mmol dicobaltoctacarbonyl and 4 mmol 3-hydroxypyridine, and pressurised to 60 bar carbon monoxide pressure. After heating for 4 hours at 75 °C, conversion of ethylene oxide was found to be virtually complete. Upon standing at room temperature for 13 hours a precipitate was formed, which was isolated by filtration and washed with methanol to yield 23 g of poly-3-hydroxypropionate, [-O-CH₂-CH₂-C(=O)-]ₙ according to ¹³C NMR.

### Example 8

Using the general procedure of Example 1, an autoclave was charged with 10 ml ethylene oxide, 40 ml methanol, 1 mmol dicobalt octacarbonyl and 4 mmol 3-hydroxypyridine, and pressurised with 60 bar carbon monoxide. After heating at 75 °C for 4 hours and analysis by GLC, 96 % of ethylene oxide was found to have been converted into methyl 3-hydroxypropionate with a selectivity of 97 %.

## Claims

1. A process for the carbonylation of epoxides by reaction with carbon monoxide at elevated pressure and temperature in the presence of a catalyst system comprising a source of cobalt and a hydroxy substituted pyridine compound.

2. A process as claimed in claim 1, wherein the hydroxy substituent is in the 3-position.

3. A process as claimed in claim 2, wherein 3-hydroxypyridine is used.

4. A process as claimed in any one or more of claims 1-3, wherein the epoxide is an 1,2-epoxide.

5. A process as claimed in claim 4, wherein 1,2-epoxide is ethylene oxide or propylene oxide.

6. A process as claimed in any one or more of claims 1-5, wherein a β-propiolactone is produced as the primary carbonylation product.

7. A process as claimed in any one or more of claims 1-5, wherein a β-propiolactone polymer is produced, by conducting the carbonylation in the presence of a polymerization promoter.

8. A process as claimed in any one or more of claims 1-5, wherein a β-propiolactone derivative is produced.

9. A process as claimed in claim 8, wherein the carbonylation is conducted in the presence of a hydroxy compound selected from water and alcohols.

10. A process for the preparation of α,β-unsaturated carboxylic acids or derivatives thereof by carbonylation of an epoxide in the presence of a cobalt-containing catalyst to form a β-propiolactone or its polymer and subsequent dehydration of the β-propiolactone or its polymer, characterised in that a catalyst system further comprising a hydroxy substituted pyridine compound is used.

## Patentansprüche

1. Verfahren zur Carbonylierung von Epoxiden durch Reaktion mit Kohlenmonoxid bei erhöhtem Druck und erhöhter Temperatur in Anwesenheit eines Katalysatorsystems, das eine Kobaltquelle und eine hydroxysubstituierte Pyridinverbindung umfaßt.

2. Verfahren nach Anspruch 1, worin der Hydroxysubstituent in der 3-Position vorliegt.

3. Verfahren nach Anspruch 2, worin 3-Hydroxypyridin verwendet wird.

4. Verfahren nach einem oder mehreren der Ansprüche 1-3, worin das Epoxid ein 1,2-Epoxid ist.

5. Verfahren nach Anspruch 4, worin das 1,2-Epoxid Ethylenoxid oder Propylenoxid ist.

6. Verfahren nach einem oder mehreren der Ansprüche 1-5, worin ein β-Propiolacton als das Carbonylierungshauptprodukt ausgebildet wird.

7. Verfahren nach einem oder mehreren der Ansprüche 1-5, worin ein β-Propiolactonpolymer ausgebildet wird, indem die Carbonylierung in Anwesenheit eines Polymerisationspromotors vorgenommen wird.

8. Verfahren nach einem oder mehreren der Ansprüche 1-5, worin ein β-Propiolactonderivat ausgebildet wird.

9. Verfahren nach Anspruch 8, worin die Carbonylierung in Anwesenheit einer unter Wasser und Alkoholen ausgewählten Hydroxyverbindung ausgeführt wird.

10. Verfahren zur Herstellung von α,β-ungesättigten Carbonsäuren oder deren Derivaten durch Carbonylierung eines Epoxids in Anwesenheit eines kobalthältigen Katalysators zur Ausbildung eines β-Propiolactons oder dessen Polymers und anschließende Dehydratisierung des β-Propiolactons oder dessen Polymers, dadurch gekennzeichnet, daß ein Katalysatorsystem, das zusätzlich eine hydroxysubstituierte Pyridinverbindung umfaßt, verwendet wird.

## Revendications

1. Procédé de carbonylation d'époxydes par réaction avec le monoxyde de carbone à pression et température élevées, en présence d'un système catalytique comprenant une source de cobalt et un composé de pyridine à substitution hydroxylique.

2. Procédé suivant la revendication 1, caractérisé en ce que le substituant du type hydroxyle se trouve en position 3.

3. Procédé suivant la revendication 2, caractérisé en ce que l'on utilise la 3-hydroxypyridine.

4. Procédé suivant une ou plusieurs des revendications 1 à 3, caractérisé en ce que l'époxyde est un 1,2-époxyde.

5. Procédé suivant la revendication 4, caractérisé en ce que le 1,2-époxyde est l'oxyde d'éthylène ou l'oxyde de propylène.

6. Procédé suivant une ou plusieurs des revendications 1 à 5, caractérisé en ce qu'une β-propiolactone est produite à titre de produit de carbonylation principal.

7. Procédé suivant une ou plusieurs des revendications 1 à 5, caractérisé en ce qu'un polymère de β-propiolactone est produit, en procédant à la carbonylation en présence d'un promoteur de polymérisation.

8. Procédé suivant une ou plusieurs des revendications 1 à 5, caractérisé en ce qu'un dérivé de β-propiolactone est produit.

9. Procédé suivant la revendication 8, caractérisé en ce que l'on entreprend la carbonylation en présence d'un composé hydroxylé choisi parmi l'eau et les alcools.

10. Procédé de préparation d'acides carboxyliques α,β-insaturés ou de dérivés de ceux-ci par la carbonylation d'un époxyde, en présence d'un catalyseur contenant du cobalt pour former une β-propiolactone ou son polymère et la déshydratation subséquente de la β-propiolactone ou de son polymère, caractérisé en ce que l'on utilise un système catalytique qui comprend en outre un composé de pyridine à substitution hydroxylique.
